# EUROPEAN PATENT APPLICATION

(11) **EP 3 275 428 A1**
(43) Date of publication of application: **31.01.2018**
(21) Application number: 16771153.0
(22) Date of filing: 22.03.2016
(51) Int. Cl.: A61K 8/97, A61K 9/08, A61K 9/107, A61K 36/61, A61Q 5/00, A61P 7/02

(54) **PEDICULICIDE FORMULATION BASED ONEUCALYPTUS GLOBULUS ESSENTIAL OIL**

(30) Priority: 27.03.2015 CL 2015775
(71) Applicant: Universidad De Concepcion, Concepción (CL)
(72) Inventor: AVELLO LORCA, Marcia Andrea, Concepción (CL); PASTENE NAVARRETE, Edgar Rafael, Concepción (CL)
(74) Representative: Carvajal y Urquijo, Isabel
(86) International application number: PCT/CL2016/000015
(87) International publication number: WO 2016/154766

(57) **Abstract**

This technology corresponds to a pediculicide formulation based on *Eucalyptus globulus* essential oil, which is effective against *P. humanus capitis,* which presents ovicidal activity and activity against *P. humanus* egg adherence, which causes the death of the parasite in a short estimated time, which has no toxicity, and which makes it impossible for the parasite to develop resistance. The formulation is composed of ethanol as a solvent and of isopropanol as a vehicle, *Eucalyptus globulus* essential oil as the active compound, optionally isopropyl myristate as an emollient, in addition to distilled water in sufficient quantity. The formulation is presented in the form of a hydroalcoholic or w/o emulsion-type lotion, if isopropyl myristate is used.

## Description

### TECHNICAL SECTOR

The technology described is destined for the health and cosmetics sector and corresponds to an innovative formulation with a pediculicidal effect based on plant extracts that are safe with low risk of generating resistance on the part of the parasite, which helps to optimise treatment of *Pediculosis capitis.*

### PREVIOUS TECHNIQUES

*Pediculosis capitis* is an international public health problem that has affected humanity throughout history. It has become widespread in the last few decades, being one of the most frequent parasitic infections in childhood. At the present time in Chile it affects more than 15% of the general population and more than 30% of children, principally in schools.

The etiologic agent *Pediculus capitis humanus* is an insect that lives on the scalp and hair of humans. Its reproductive cycle is complex, which makes it more difficult to eradicate. Collateral effects such as skin abrasions, as a consequence of scratching, and subsequent infection make this a multifactorial pathology.

It also has a significant economic impact due to the relatively high cost of treatment, to which must be added laundry costs in the home and/or at a commercial laundry, in addition to the time spent on such tasks. From an emotional perspective, the family suffers because of the belief that head lice proliferate in a dirty house. Furthermore, school children are often sent home and not allowed to return to classes until they are free of the parasites, which leads to a feeling of shame in the family and frequent social rejection by friends and neighbours. At the same time, head lice are associated with anxiety and fear (Cazorla et al. 2007).

The parasite is present throughout the year, but an increase is observed during the first month after the beginning of the school year. Its appearance is not necessarily associated with poor hygiene, as the insect actually prefers to colonise clean hair (See *¿Problemas con los piojos?* [Problems with lice?] *Informative newsletter).* It is most commonly found in urban and suburban areas, especially those with high concentrations of population. The infection rate of men to women is 1:2.

At the present time it is estimated that there is a global rise in the prevalence of *Pediculosis capitis,* resulting from the lack of effectiveness of existing pediculicides, in addition to an increase in resistance to the products currently available.

Insecticides used in Chile to treat pediculosis include some pyrethroids and pyrethrins, such as permethrin and decamethrin, and also crotamiton and thiabendazole, although the latter are mainly used as scabicides to treat scabies. In some countries it is common to periodically rotate the use of medicines for *P. capitis* treatment in order to reduce the occurrence of resistance. This problem has been observed with both new pyrethroids and with older insecticides such as lindane, although the effectiveness of these rotation practices is disputed.

Lindane (gamma benzene hexachloride) is used against *P. capitis* in a 1% solution, being applied topically for 6 to 24 horas, with a repeated application a week later, since it lacks ovicidal activity. Its topical use can provoke local hypersensitivity reactions and in serious acute cases it may damage the central nervous system. Approximately 10% is absorbed by passage into the blood stream, where its average life is 24 hours; it is eliminated mainly by the kidneys. The absorption rate is higher in small children. The toxicity range in adults is 28 g, which can be lethal, but it has been reported that ingestions of 45 mg can already cause systemic damage; in children below the age of 4 a dose of 5 ml at 1% can cause respiratory depression.

In Chile, taking into account the background to the 2009 Supreme Decree N°54 from the Ministry of Health prohibiting the use of lindane or hexachlorocyclohexane in pesticides for sanitary and household use and in pharmaceuticals, and taking into consideration the fact that the use of lindane in pharmaceuticals could be toxic, affecting the health of the user, it was decided to withdraw the health registration of pharmaceutical products that contain this active principle on March 5, 2012. (B11/Ref: 3385/11 - Exempt Resolution 601).

Among pyrethroid derivatives we can highlight decamethrin 0.2%, which is generally used in two applications 7 days apart. Numerous cases of sensitisation and contact dermatitis, associated with abrasions, have been reported, in addition to numerous cases of resistance. Permethrin (synthetic pyrethroid) has a neurotoxic effect for *P. humanus, Pthirus pubis* and *Sarcoptes scabiei.* It is used in a 1%-3% solution. Its pediculicidal activity lasts for 10 to 14 days, which would make it possible to use only one dose; however, experience shows better results after a second dose 7 days after the initial treatment, and currently three applications 2 days apart are being recommended because of the parasite's resistance to this and other pyrethroids. Pyrethrins (0.02-5%), are topically-used substances extracted from chrysanthemums and incorporated into a shampoo. As they have no ovicidal activity, the application must be repeated 7 days later. Pyrethrins appear to be as effective as or more effective than lindane, although there have been many cases of resistance reported. Allergic reactions have also appeared in sensitive patients, including systemic allergic reactions. Their inhalation can provoke a reaction of hypersensitivity of the airways. Their ingestion can produce fatigue, headaches, anorexia, nausea and vomiting. The ingestion of large amounts (200-500 mL) of concentrated formulas could quickly lead to a state of coma. Cardiovascular, neurological and immunological effects have been reported.

Crotamiton (N-ethyl-0-crotonotoluidide) is a pediculicide and scabicide administered topically and dispensed in a cream or lotion at a concentration of 10% that also contains oxyquinoline sulfate, the preparations generally having antipruritic properties. The main adverse effect of its use is contact dermatitis, especially in previously inflamed skin, or when it is applied for a prolonged period of time. There are no conclusive studies on its percutaneous absorption. Its ingestion could cause drowsiness, nausea, vomiting, hypotension, general malaise, and even a state of coma with hyperreflexia. Thiabendazole derives from substituted compounds of benzimidazole, some of which show total larvicidal activity *in vitro,* which, added to the absence of activity against other microorganisms and its relatively low toxicity for mammals, means that its use is recommended against some parasites such as cutaneous larva migrans or trichinosis. It can be applied to the skin in a 10% suspension for the treatment of scabies or pediculosis, although there are no studies that support its efficacy. Thiabendazole is absorbed percutaneously, being eliminated, with an average life of 70 minutes.

In addition, new pediculicides have been developed, such as Levamisol, an agonist of the nicotinic receptors for acetylcholine that is rapidly and almost completely absorbed through the gastrointestinal tract. It is highly effective in eradicating ascaris and trichostrongylus. A study conducted by Namazi, (2001) demonstrated the effectiveness of this pharmaceutical in the treatment of pediculosis. Unfortunately, its effectiveness is low (19%), which could be due to the resistance of the parasite to the drug caused by its widespread use in the treatment of intestinal parasitosis in the area of study, in which many children had both intestinal parasites and head lice.

Aliphatic lactones are found in many fruits and play an important role in providing flavour. In a study carried out in Argentina, Toloza et al. (2006) evaluated the fumigant and repellent activity of three alphatic lactones against *Pediculus humanus capitis* resistant to permethrin. Their results did not show fumigant activity in the alphatic lactones, but they did find significant repellent activity with α-dodelactone.

Various essential oils derived from plants have been studied as pediculicides, as they have been widely used in traditional medicine to eradicate lice. These oils are composed of numerous terpenes, are highly volatile, and have a low molecular weight. Yang et al. (2005) studied the pediculicidal effect of *Cinnamonum zeylanicum* (cinnamon) and found that it had some such effect, but they concluded that more studies were needed on both the safety of its use in humans and on pharmaceutical formulations that could improve the effectiveness of the compounds and their stability, thereby reducing costs.

Other studies have focused on the pediculicidal activity of eucalyptus essential oil. Toloza et al. (2010) concluded that various eucalyptus essential oils presented strong fumigant activity against lice resistant to permethrin. Yang et al. (2004) established that the pediculicidal compounds in the essential oil from *Eucalyptus globulus* leaves, such as 1.8-cineol, (-) α-pinene, 2-α-pinene, (*E*)-pinocarveol, /phellandrene, α-terpinene, and 1-α-terpineol were as effective as ð-phenothrin or pyrethrum, two commonly-used pediculicides. Another essential oil studied as a pediculicide is that obtained from *Eugenia caryophyllata* (Chilean avens), eugenol being one of its main compounds. This study showed that the efficacy of the essential oil was comparable to that of commercial products based on -phenothrin and pyrethrum, but far lower than that of pyrethroids. Oregano essential oil has also been an object of study. Yang et al. (2009) studied the pediculicidal activity of essential oils from *Origanum majorana* (oregano) on *Pediculus humanus capitis* resistant to pyrethroids and to malathion. Their results indicate that some of the components of this essential oil are just as effective as commercial pediculicides based on α-phenothrin and pyrethrum, some having more rapid action than the latter, and others showing less or no pediculicidal activity.

In general, the authors of all the studies conducted on essential oils from plants suggest more research on product safety for humans and more research to improve pediculicidal efficacy and the stability of the formulation.

Heukelbach et al. (2006) evaluated the efficacy of a commercial pediculicidal shampoo based on extracts of *Azadirachta indica* (Neem tree), using *in vitro* studies. The death of all the parasites occurred after three hours of immersion in the shampoo.

Dimethicone causes the death of the parasite through a physical mechanism, by interrupting the entry of oxygen into the insect in general, and is a good alternative to classic pediculicides. The good results observed are produced by using high concentrations of dimethicone or if the individual concerned has only a moderate infestation.

At the present time, the formulations sold in Chile have active ingredients shown to be only partially effective in the treatment of pediculosis and these ingredients have developed the resistance of the parasites to their pharmacological activity. These actives are incorporated into formulations of the types lotion with rinse, washable cream and shampoos. Additionally they use organic acids as coadjuvants for conventional treatments to enhance the anti-adherence effect on early stages of the parasite.

The following table shows the principal agents used in the treatment of *Pediculus humanus* in Chile and their current clinical situation.

**Table 1: Principal agents used in the treatment of Pediculus humanus in Chile and their current clinical situation.**

| **PRODUCT** | **CATEGORY** | **MODE OF USE** | **TOXICITY** | **COMMENTS** |
|---|---|---|---|---|
| **Decamethrin** Lotion 20% (Derivative Pyrethroid) | Pediculicide Larvicide | Two applications, repeated one week later | Local hypersensitivity. Neurotoxic | Development of resistance |
| **Permethrin** Lotion and Shampoo 1-3% (Derivative Pyrethroid) | Pediculicide Larvicide | Three applications in one week. | Local hypersensiitivity. Neurotoxic | Development of resistance. More effective than lindane |
| **Pyrethrin** Shampoo 0,02-5% (Piperonyl butoxide, association) (Derivative Pyrethroid) | Pediculicide Larvicide | One application, repeated one week later. | Local and systemic hypersensitivity. Neurotoxic | Development of resistance |
| **Crotamiton** Lotion 10% (Oxyquinoline sulfate, | Pediculicide Larvicide (Coadjuvant) (Used more | One application, repeated one week later. | Local and systemic hypersensitivity Neurotoxic | Development of resistance. |
| association) | for scabies) | | | |
| **Organic acids** (Vinegars) | Coadjuvant | Follows the application of the pediculicide that it accompanies | Not described | |

The appearance of resistance on the part of the parasite to conventional pediculicides that are currently used is a very preoccupying global problem. The costs of this pathology are both economic and social; poor diagnosis and incorrect use of pediculicidal medication have contributed to reducing the efficacy of these products. Taking this situation into consideration, it is urgent to discover new pediculicidal alternatives that are safer and more effective by introducing new treatment options.

Despite the fact that human pediculosis is a very common condition throughout the world, few research groups have tried to find new treatments. There is evidence of the pediculicide activity of volatile terpenic and phenolic compounds, usually concentrated in introduced aromatic plants, but also found in species that are widely distributed and sold in Chile. These extracts have exhibited action comparable to important pediculicides such as lindane, permethrins and with larvicidal action, yet they have low toxicity, revealing themselves to be alternative treatments against parasites that have acquired resistance to conventional pharmaceutical drugs.

Some papers mention faulty diagnosis, failure to complete treatment and/or incorrect use of medication and overdose-overexposure as possible causes of the appearance of resistance to pyrethroids, pyrethrins and lindane.

Furthermore, various essential oils obtained from plants have been studied in the search for new pediculicides, particularly those used widely in traditional medicine for the eradication of parasites. Some studies (Priestly et al., 2006) have shown that mono-oxygenated compounds that possess structures with a simple alcohol, or acetone or phenol functional groups, have been most active as pediculicides. Non-oxygenated terpenes were inactive in the conducted studies. It was also reported that some compounds presented good ovicidal activity, but no pediculicidal activity; and, on the contrary, some compounds presented only pediculicidal activity.

Yang et al. (2005) studied the pediculicidal effect of cinnamon and found evidence of some such effect, but they concluded that more studies were needed on the safety of its use in humans, in addition to research on pharmaceutical formulations that could improve the effectiveness of the compounds and their stability, thereby reducing costs. Other studies have focused on the pediculicidal activity of eucalyptus essential oil: Toloza et al. (2010) concluded that various eucalyptus essential oils presented strong fumigant activity against parasites resistant to permethrin. Yang et al. (2004) established that the pediculicidal compounds present in essential oil from *Eucalyptus globulus* leaves, such as 1.8-cineol, (-) α-pinene, 2-α-pinene, (E)-pinocarveol, I-phellandrene, α-terpinene, and 1-α-terpineol were as effective as ð-phenotrine or pyrethrum, which are two commonly-used pediculicides.

Another essential oil studied as a pediculicide is that obtained from Chilean avens, of which one of the principal compounds is eugenol. One study observed an essential oil efficacy comparable to that of commercial products based on α-phenotrine and pyrethrum, although that efficacy was significantly lower than that of pyrethroids. Oregano essential oil has also been studied. Yang et al. (2009) studied the pediculicidal activity of this essential oil on *P. humanus capitis* resistant to pyrethroids and to Malathion. Their results indicate that some of the components of this essential oil are as effective as commercial pediculicides based on α-phenotrine and pyrethrum, some with more rapid action than the latter, and others with less or no pediculicidal activity.

In general, in all the studies conducted on essential oils from plants, the authors recommend more research on product safety for humans and more research to improve pediculicidal efficacy and the stability of the formulation. The action mechanism possessed by essential oils is provided by terpenes and other components that have a fundamental antimicrobial and insecticidal role, given their lipophilic nature. This is why these components have shown interaction with the lipid barriers of microorganisms, destabilising the integrity of the membranes and other structures rich in lipopolysaccharide molecules and associated with enzymes that are capable of breaking down molecules introduced from outside. The spontaneous formation of complexes between terpenoid molecules and membrane cholesterol has been proposed, for example, which would cause an increase in the permeability of the membrane, allowing the circulation of ions and macromolecules between proteins. As a consequence, sterols would be extracted through vesicles and death would occur because of the functional failure of the invading organism.

The development of new formulations for the treatment of pediculosis is a vital challenge for the control of populations of *Pediculus humanus capitis* resistant to conventional pediculicides. What is needed are formulations that are efficacious, stable, with optimal bioavailability, good skin tolerance and free of toxic effects. Formulations used as vehicles for pediculicidal agents must possess a series of characteristics: i) They must be easy and pleasant to apply; ii) they must allow the active principle to act without any interference and extend its presence in the treatment area; iii) they must be able to protect and condition hair without causing irritation; iv) their organoleptic properties should not create a negative reaction in the product user.

### BRIEF DESCRIPTION OF THE FIGURES

**Figure 1****:** Chromatogram eucalyptus essential oil.
**Figure 2****:** Pediculicidal activity *in vitro* of the different essential oils.
**Figure 3****:** Toxicity in the vapour phase of the final product.
**Figure 4****:** Toxicity of the final product on lice through immersion.

### DISCLOSURE OF THE INVENTION

The present technology corresponds to an innovative formulation, with a pediculicidal effect based on plant extracts that is safe and with a low risk of generating resistance in the parasite, which helps to optimise the treatment of *Pediculosis capitis.*

The product is effective on *P. humanus capitis,* presents ovicidal activity and activity on *P. humanus* egg adherence, causes an estimated rapid death, has no toxicity, makes it impossible for the parasite to develop resistance, its raw material is widely available, and it is an organic product whose processing is environmentally friendly, which set of characteristics distinguish it from the products currently on the market.

This formulation has demonstrated action superior to that of well-known pediculicides such as lindane and permethrins, and in addition it presents larvicidal action, yet with low toxicity, so that it can be presented as an alternative treatment against parasites that have developed resistance to conventional pharmaceuticals.

This formulation corresponds to a hydroalcoholic lotion that contains *Eucalyptus globulus* essential oil. The composition of the lotion is described in Table 2:

**Table 2: Lotion composition.**

| **Raw material** | **Concentration (% v/v)** |
|---|---|
| Ethanol 96° | 20 - 35 % |
| Isopropanol | 20 - 35 % |
| *Eucalyptus globulus* essential oil | 1-10% |
| | |
| **Optionally** Isopropyl myristate | 35-45% |
| Distilled water | Sufficient quantity |

Where ethanol is the formulation solvent, isopropanol is the vehicle, *Eucalyptus globulus* essential oil is the active compound, isopropyl myristate is used as an emollient, and the formulation is completed with a sufficient quantity of distilled water. The lotion containing isopropyl myristate looks like a w/o emulsion.

The presentation of the formulation in a lotion means that it can be used efficiently in areas of high hair density. This liquid pharmaceutical form prolongs contact time between the pediculicidal preparation and the parasite in comparison with other forms of application, thus allowing greater penetration and more residual activity and conferring an ovicidal effect on the formulation that is more powerful than that of creams. These characteristics make lotions the ideal choice for the treatment of the parasitic infection in question.

The following are some differences in comparison with solutions similar to the new product:
- 100% increase in ovicidal activity compared with current products.
- 100% effective against egg adherence.
- Impossibility of the parasite developing resistance.
- Short estimated time to death (1-3 minutes).
- Toxicity close to 0.

The eucalyptus essential oil used in the formulation is obtained from raw material from forestry activity, implying low costs, as the leaves are considered to be residues. The extraction process basically consists in hydrodistillation, but extraction can also be carried out using CO₂ supercritical fluids

The formulation conforms to routine controls and maintains its physico-organoleptic characteristics and the concentration of the chemical marker chosen for at least the 180 days of the study.

This formulation produces death in adults and young individuals within a time period that varies between 1 and 2 minutes, in contrast to the pediculicide Launol® (control), which immobilised the parasites for 10 minutes, after which time they revived.

Due to its action mechanism against *Pediculosis capitis,* this formulation can also be used against other ecto-parasitic infections such as:
- Pediculosis from *Pediculus humanus vestimentis* (clothing lice).
- Pubic pediculosis from *Phthirus pubis* (crab lice).
- Scabies from *Sarcoptes scabiei.*
- Infestations from Ixodes such as the superfamily *Lxodidae* (ticks).

The formulation can also be used as a mosquito repellent.

### APPLICATION EXAMPLES

### Example 1: Method of obtaining the essential oil and its characterisation.

*Eucalyptus globulus* essential oil was obtained through hydrodistillation in a Clevenger apparatus. Approximately 800 grammes of leaves and aerial parts of fresh plants were immersed in 5 litres of water in a Florence flask for at least 12 hours. This was then brought to boiling point for 90 minutes, using an electromantle that was directly in contact with the flask. The essential oil obtained was dried with anhydrous sodium sulfate and was stored in refrigeration at 4°C in a previously-labelled amber container, until it was used. The yield was determined in ml of essential oil obtained from each 100 grammes of plant used.

To characterise the extract, 15 µL of essential oil were dissolved in 10 ml of absolute ethanol to be analysed using gas chromatography under the following conditions:
- Perkin Elmer gas chromatograph Clarus 500.
- Injector temperature: 260°C
- Column temperature: gradient 80 - 130°C
- Detector temperature: 240°C
- Column: Phenyl methyl silicone, 30 m x 0,53 mm x 1.0 µm
- Detector: Flame ionisation (FID)

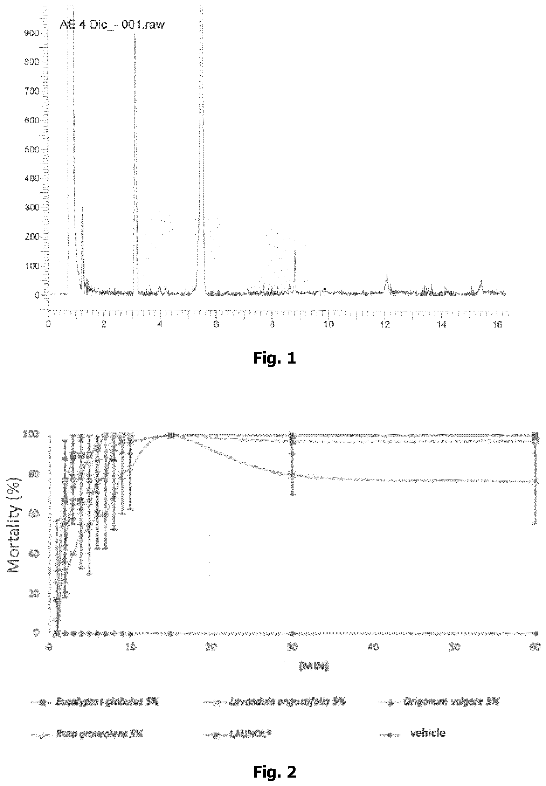

The chromatographic analysis produced a profile with the appearance of 4-5 main compounds (Figure 1, Table 3).

**Table 3: Compounds identified in eucalyptus essential oil and their percentage.**

| **Compound** | **Percentage** |
|---|---|
| α-pinene | 0,05-10,0 |
| β-pinene | 0,05-1,5 |
| Sabinene | maximum 0,3 |
| α-phellandrene | 0,05-1,5 |
| Limonene | 0,05-15,0 |
| 1,8 cineol | minimum 70,0 |
| Camphor | maximum 0,1 |

### Example 2: Bioassays and in vitro toxicity studies of essential oils.

To ascertain the efficacy of the extract actives against *Pediculus humanus, in vitro* tests were conducted on *Eucalyptus globulus* (eucalyptus), *Lavandula angustifolia* (lavender, *Rosmarinus officinalis* (rosemary), and *Origanum vulgare* (oregano). An average of 4 young and adult parasites were placed in glass Petri dishes with human hair from the location of the parasites. They were exposed to varying dilutions of different extracts in alcohol-water mixtures. The mortality of the parasites was evaluated each minute to observe changes over a total period of one hour. Adults were considered dead when their appendices stopped moving when stimulated by a swab or there was loss of the straightening reflex. Launol® was used as a control substance.

Table 4 shows the activity of different essential oils against parasites and the time that adult individuals took to die in different dilutions. The best results, death after 1 or 2 minutes, were observed with *Eucalyptus globulus* and *Lavandula angustifolia* in concentrations of 3 to 5% for eucalyptus and 4 to 10% for lavender. Concentrations of up to 10% of *Rosmarinus officinalis* and *Origanum vulgare* caused death in less than 2 minutes in adults.

**Table 4: Time to death caused by different essential oils.**

| **Essential Oil** | **Concentration** | **Total n° of adult specimens** | **Specimens** | **Time to death (minutes)** |
|---|---|---|---|---|
| | | | 1 | 1 |
| Rosemary | 10% | 3 | 1 | 2 |
| | | | 1 | 6 |
| Oregano | 5% | 10 | 6 | 2 |
| | | | 3 | 3 |
| | | | 1 | 6 |
| | 10% | 1 | 1 | 1,5 |
| Eucalyptus | 4% | 4 | 2 | 1,5 |
| | | | 2 | 2,5 |
| | 5% | 11 | 4 | 1 |
| | | | 2 | 2 |
| | | | 5 | 3 |
| Lavender | 4% | 6 | 3 | 1,5 |
| | | | 1 | 3 |
| | | | 2 | 3,5 |
| | 10% | 4 | 4 | 1 |

| | | | | |
|---|---|---|---|---|
| All essential oils were dissolved in EtOH 50% (100 µl) | | | | |

The control product used (Launol®) attenuated the reactions of the parasites, immobilising them for 10 minutes, after which time they revived. This did not occur in the essential oil dilutions.

For the next tests essential oil concentrations of 5% were used, in order to compare their activity against different stages of *Pediculosis capitis.*

**Collection of specimens:** For the sample, *P. capitis* adults and eggs were collected from the heads of children aged between 5 and 14 from primary schools in different sectors of the City Concepción.

These children diagnosed with pediculosis were invited to participate in the study with the previous authorisation of their parents, who signed an informed consent form. Children who had received any type of treatment for this infection during at least the previous month were excluded.

The lice were collected using fine-toothed metal combs specially designed to remove lice eggs and transported to the laboratory in Petri dishes following the protocol established by Picollo et al. (1998, 2000) over a maximum period of 2 hours. After examination under a microscope, damaged specimens were discarded. To ensure maximum survival, the selected specimens were kept in the dark at 18°C with 70-80% relative humidity to reduce the dehydration suffered by the insect when taken out of its micro-habitat.

As mentioned, the eggs were collected with a fine-toothed comb or by cutting the hair with scissors. Eggs found at less than 1 cm from the scalp were chosen. After collection they were taken to the laboratory, where they were separated according to their stage of development and immediately used in the establishment and study of colonies.

Heparinised blood from donors was used to establish the *P. humanus capitis* colonies. One volume of this blood (1 mL) was adsorbed in a cotton ball with a diameter of 35 mm. Then the cotton ball was isolated between two sheets of parafilm that had previously been stretched to create a sheet with a thickness of about 0.1 mm. This blood pad was trimmed and then deposited in a Petri dish 90 mm in diameter. The Petri dish was covered with a fine mesh to allow ventilation and placed on a heating plate adjusted to 37°C, where it was left to equilibrate for a few minutes. When they were not feeding, adults were kept at 75% relative humidity in Petri dishes at 30°C, with a piece of black felt to allow them to move around easily. This piece of felt 2 cm in diameter holding the lice was placed daily on the Petri dish that contained the pad in the pre-heated system. The lice were permitted to feed for 15 minutes. This setup made it possible to maintain the adult colonies for at least one month. The survival of adult individuals and the viable eggs (nits) laid were evaluated periodically. The adult lice and nymphs in good condition were selected.
**Evaluation Criteria for Lethality:** Adult mortality was evaluated every 5 minutes for 5 hours. Adults were considered dead if their appendices did not move when stimulated with a wooden stick or there was loss of straightening reflex. In the case of eggs, the evaluation was based on the number of unhatched eggs 12 days after treatment. Pyrethrins and lindane acted as positive controls. The average values of lethality time (LT₅₀) were calculated using a probit analysis.
**Pediculicidal Activity *in vitro:*** To evaluate lethality time for adults, a bio-assay involving contact between the parasite and the compounds deposited on a paper filter disc was used. Females were exposed to different concentrations (in mg/cm²) of the substances dissolved in acetone and applied to paper filter discs N°2 2,5 cm in diameter. Discs impregnated only with acetone were used as a control.

After the discs were dried under an extraction hood for 2 minutes, each one was placed on the bottom of a Petri dish. Groups of 20 females (7-9 days old), fed on human blood 4 hours before the assay, were placed in each Petri dish. Then some strands of human hair were added and they were closed for incubation at 18°C and 70-80% relative humidity.

Figure 2 presents the graph of the pediculicidal activity, showing that eucalyptus essential oil acts faster than the reference product Launol®. In addition, almost 100% effectivity is obtained with this concentration for 3 of the essential oils except that of *Lavandula angustifolia.*
**Ovicidal Activity *in vitro:*** To evaluate lethality time for *P. humanus capitis* eggs, different concentrations (in mg/cm²) of the samples were tested by being dissolved in acetone and applied on filter paper discs. The filter papers used as a control only received acetone. After drying for 2 minutes, the eggs (3-4 days old) adhering to the hair strands were placed on the treated filter papers in each Petri dish and the lid was kept on for 24 hours (18°C/70-80%). The hatching inhibition percentage (HIP) was calculated using the formula: HIP (%) = [(C-T)/C] x100, where C is the hatching percentage of the control and T is the hatching percentage of the treatments.

The HIP of the eucalyptus essential oil was 100%, while those of the oregano essential oil and the rue essential oil were both 80%.

On the basis of the indicated results, a formulation using *Eucalyptus globulus* essential oil at 5% was created, corresponding to the oil with the best results.

### Example 3: Development and characterisation of the formulation.

In order to characterise the efficacy of the technology, two types of formulations with *Eucalyptus globulus* essential oil at 5% were prepared (Table 5), one of them including isopropyl myristate serving as an emollient (Table 6). These formulations were presented in the form of a lotion, which gives them optimal properties when applied, facilitating administration.

**Table 5: Hydroalcoholic Lotion.**

| **Raw material** | **Concentration (% v/v)** |
|---|---|
| Ethanol 96° | 32,5% |
| Isopropanol | 32,5% |
| *Eucalyptus globulus* essential oil | 5% |
| Distilled water | 30% |

**Table 6: Emulsion Type o/w Lotion.**

| **Raw material** | **Concentration (%v/v)** |
|---|---|
| Ethanol 96° | 25% |
| Isopropanol | 25% |
| *Eucalyptus globulus* essential oil | 5% |
| Isopropyl myristate | 40% |
| Distilled water | 5% |

*In vitro* controls were carried out on the formulation, such as:
- Viscosity: A Brookfield viscometer was used to measure the viscosity of stored samples at 0, 30, 60, 90 and 180 days.
- Water loss through evaporation: This is frequent in preparations containing volatile compounds. It was measured in stored samples at 0, 30, 60, 90 and 180 days.
- Size and distribution of particle size: The drop size for emulsion type lotions was determined using optical microscopy for the stored samples at 0, 30, 60, 90 and 180 days. Changes in the size and size distribution of drops may be indicative of physical instability processes such as flocculation and coalescence.
- Quantity and release of active principles using Franz cells.
- Formulation stability studies: An accelerated stability study was conducted for six months on a minimum of three series of the product in its final form at a temperature of 40°C ± 2°C with a relative humidity of 75% ± 5% in a climatic chamber according to the norms of the International Conference on Harmonisation (ICH). The potency (chemical stability) and physical characteristics of the product were monitored. The concentration of a chemical marker was evaluated at 0, 30, 90 and 180 days to determine if it was within acceptable limits. A change of 5% in the initial concentration of the active principle constitutes a significant change in the product, as does a degradation product found in quantities beyond the acceptation criterion, and changes relating to the acceptation criteria of the product, such as physical attributes (appearance) and functionality test (colour, separation of phases, hardness, etc.).

The formulations passed routine checks and maintained their physico-organoleptic characteristics and the concentration of the chemical marker chosen (1,8-cineol, 4476-45%) for the period of the study, both in real time conditions and in accelerated conditions.

### Example 4: Bioassays and in vitro Toxicity Studies on the Pediculicidal Formulations.

**Toxicity in the Vapour Stage of the Final Product:** The fumigant activity of the formulations against *P. humanus capitis* females was investigated according to the method of Yang et al. (2003). Groups of 20 females (7-9 days old) were placed on the bottom of a Petri dish and covered with a fine wire mesh, 4,7 cm in diameter. Each filter paper (5 cm in diameter), treated with the substances (mg/cm²) dissolved in acetone, was placed over the wire mesh (18°C with 70-80%). This prevented direct contact between the females and the compounds being tested. In order to investigate toxicity in the vapour stage, another cover was placed on each Petri dish. The control consisted of filter paper impregnated only with acetone.

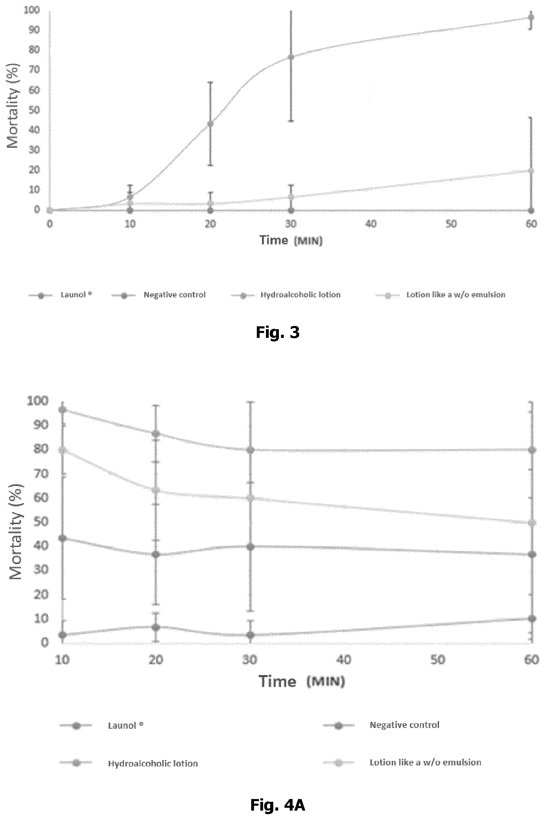

Figure 3 is a graph showing the toxicity of the final product in which we can see the potent fumigant activity of the hydroalcoholic lotion with eucalyptus, with no significant statistical differences between the fumigant activity of the o/w emulsion type lotion, the negative control and Launol®.
**Toxicity Test on Lice by Immersion in the Final Product:** A toxicity test by immersion was used to evaluate the effectiveness of the final product. For this purpose, the lice adhering to the hair strands were completely immersed in the products for 3 minutes. Subsequently, the females were dried on filter paper. After the remaining product was removed, the females were transferred to a metal mesh and washed under running water to simulate application on an infested patient. Once washed, the lice were placed on Whatman N°1 filter paper and left for a recovery period of 10 minutes, during which the lice inactivated by immersion but not poisoned recover their normal activity (reversal). The test was applied to about 50 females whose activity was evaluated at 5, 10, 15, 30, 45, 60, 90 minutes and 18 hours (18°C with 70-80%). The louse was considered to be dead if it was incapable of walking over filter paper, remained on its back, and did not move its antennae or legs. The viability criteria made it possible to classify the activity as follows: 1) A totally active individual with normal movements; 2) an individual with major vital signs, but not able to move forward or recover its straightening reflex; 3) individuals with reduced vital signs or those that only present slight internal movement (stomach), minimal movement of the antennae or legs when stimulated with forceps; and, finally, 4) individuals without vital signs that do not respond to stimulation with forceps.
A control group consisted of insects immersed in water. The data on recovery at 10 minutes and mortality at 18 horas were processed according to ANOVA followed by the Tukey Test.

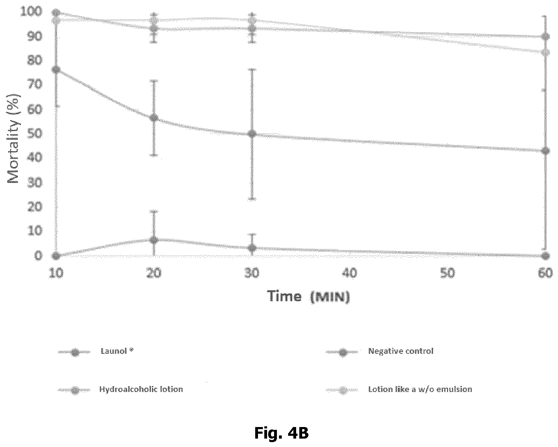

The mortality percentage was determined according to Mougabure Cueto et al. (2002), as charted in Figure 4, for 10 minutes of immersion (Fig. 4A) and 15 minutes of immersion (Fig. 4B). In both cases, the controls presented fewer and statistically different actions from those of the hydroalcoholic o/w emulsion type lotions; however, the highest mortality percentage was obtained after an exposure of 15 minutes.

Groups of 10 to 20 viable eggs in late development stages were used for the ovicidal effect test. These eggs had black eye points and the embryo appendage was clearly visible. They were attached to a slide with two-sided adhesive tape, and the slide and eggs were immersed in the new products for 0,5; 1, or 2 minutes.

The mortality of the treated eggs (100%) was recorded 5 days after the hatching of the controls. The mortality criterion for the eggs was eggs that did not hatch or eggs that hatched with the nymph inside. The eggs were incubated in optimal conditions in a chamber at 27-31°C and with 45-75% humidity.
**Bioassays on the Tissue Toxicity of the Final Product:** One of the important parameters to evaluate was safety at the time of local application to the scalp. To this purpose, the HET-CAM test was conducted to determine the level of irritation caused by the compound or preparation in use. Fertilised hens eggs were incubated and opened carefully, exposing the chorion allantois membrane. 300 mg or 200-300 µl of the products under study were placed on this membrane. Up to 5 minutes after the application the membrane was observed for signs of intravascular clotting, blood vessel lysis, haemorrhage or vasodilatation. No irritation was observed with either of the two formulations tested.

### Example 5: Prospective, Randomised, Open Clinical Trial

A clinical study was carried out on 10 participants who were children infested with lice aged between 5 and 15 from schools in the city of Concepcion. These participants were selected after a 3-minute visual inspection or if they were found with 24 lice on their scalp.

The following were the participant exclusion criteria: use of pediculicidal, antihelminthic or antibiotic products in the four weeks preceding treatment; severe scalp disorders; cosmetic hair treatment in the four weeks preceding treatment; sensitivity to any ingredient in the product; or mental illness.

The parents or guardians of the infested children were invited to attend an informative talk and only children with the written consent of the adults responsible for them were included in the study. These adults were given instructions on how to apply the product.

At the same time, the adults were asked to clean the personal belongings of the participants and the people in direct contact with them, including clothes and toilet articles, with boiling water or dry heat.

The participants received the first application for 30 minutes, and this process was repeated 7 days later (the two formulations (n=4 and 4). The commercial product launol® was used in the same way for the control group (n=2).

The products were applied to dry skin and were spread generously all over the patient's scalp, covering hair roots and paying special attention to the areas behind the ears and to the neck. After the aforementioned exposure time, the scalp was washed with plenty of warm water. Subsequently, the patient's hair was combed to extract the nits for observation in the laboratory.

An inspection was carried out on the second and seventh days of treatment, and then two days after the end of treatment. The treatment was considered effective if there were no live lice on the scalp, when damp, one or two days after the first application and absolutely no eggs two days after the end of treatment. The treatment was considered defective with a score equal to or greater than 1.

To classify the results of this study, they were given a score according to the number of specimens found during the evaluation period, as shown in Table 7:

**Table 7: Score allocation according to number of specimens found.**

| **Classification** | **Number of specimens** | **Allocated score** |
|---|---|---|
| No nits found | 0 | 0 |
| Light infestation | < 10 nits | 1 |
| Moderate infestation | Between 10 and 20 nits | 2 |
| Severe infestation | > 20 nits | 3 |

**Level of cure:** The percentage of patients cured after the application of the treatment was 100%, with a score of 0 for both formulations. The control group had a score of 3.

The level of itching was recorded before, during, and after treatment, using a visual analogue scale in which redness, dryness, irritation and skin peeling were evaluated: a score of 0 = absence; 1=light; 2=moderate; 3=severe. With regard to all parameters, both formulations received a total score of 0, as did the group that used the commercial product.

The cosmetic acceptability of the product was evaluated using a scoring summary and a standard questionnaire that included organoleptic characteristics, scalp irritation and cosmetic hair changes. A total score of 0 was obtained by both formulations; where the measuring scale of discomfort, irritation and itching was: 0=absence, 1=light, 2=moderate, 3=severe. The control group also obtained a score of 0.

## Claims

1. A pediculicide formulation **CHARACTERISED by** the fact that it is composed of:
a. 20 - 35 % Ethanol 96°,
b. 20 - 35 % Isopropanol,
c. 1 - 10 % Essential oil of *Eucalyptus globulus,* and
d. Distilled water in sufficient quantity.
Optionally
e. 35 - 45 % Isopropyl myristate.

2. A pediculicide formulation, according to Claim 1, **CHARACTERISED by** the fact that ethanol acts as a solvent, isopropanol is the vehicle, the active compound is *Eucalyptus globulus* essential oil, and isopropyl myristate is used as an emollient.

3. A pediculicide formulation, according to Claim 1, **CHARACTERISED by** the fact that it is a hydroalcoholic lotion.

4. A pediculicide formulation, according to Claim 1, **CHARACTERISED by** the fact that the lotion containing isopropyl myristate looks like a w/o emulsion.

5. A pediculicide formulation, according to Claim 1, **CHARACTERISED by** the fact that it can be easily and efficiently applied in areas of high hair density.

6. A pediculicide formulation, according to Claim 1, **CHARACTERISED by** the fact that it is safe.

7. A pediculicide formulation, according to Claim 1, **CHARACTERISED by** the fact that it allows more contact time between the pediculicidal preparation and the parasite than other forms of application.

8. A pediculicide formulation, according to Claim 1, **CHARACTERISED by** the fact that it maintains its physico-organoleptic characteristics at least for the 180 days of the study.

9. Use of the pediculicide formulation, according to Claim 1, **CHARACTERISED by** the fact that it is effective against *P. humanus capitis.*

10. Use of the pediculicide formulation, according to Claim 1, **CHARACTERISED by** the fact that it presents ovicidal activity and activity against *P. humanus* egg adherence.

11. Use of the pediculicide formulation, according to Claim 1, **CHARACTERISED by** the fact that it causes the death of the parasite in an estimated time of between 1 and 3 minutes, and makes it impossible for the parasite to develop resistance.

12. Use of the pediculicide formulation, according to Claim 1, **CHARACTERISED by** the fact that it offers alternative treatments against parasites that have developed resistance to conventional pharmaceuticals.

13. Use of the pediculicide formulation, according to Claim 1, **CHARACTERISED by** the fact that, due to its action mechanism against *Pediculosis capitis,* this formulation can also be used against other ectoparasitosis such as:
a. Pediculosis from *Pediculus humanus vestimentis* (clothes louse),
b. Pubic pediculosis from *Phthirus pubis* (crab louse),
c. Scabies from *Sarcoptes scabiei*; and
d. Infestation from Ixodes, such as the superfamily *Lxodidae* (hard tick).

14. Use of the pediculicide formulation, according to Claim 1, **CHARACTERISED by** the fact that it is used as a mosquito repellent.
